# EUROPÄISCHE PATENTSCHRIFT

(11) **EP 0 694 071 B1**
(45) Veröffentlichungstag und Bekanntmachung des Hinweises auf die Patenterteilung: **06.03.2002**
(21) Anmeldenummer: 94913539.6
(22) Anmeldetag: 06.04.1994
(51) Int. Cl.: C12N 15/87, C12N 15/86, C12N 7/04, A61K 48/00

(54) **ADENOVIRUS FÜR DEN TRANSPORT VON FREMD-DNA IN HÖHERE EUKARIOTISCHE ZELLEN**
ADENOVIRUS FOR THE TRANSFER OF FOREIGN DNA INTO HIGHER EUCARYOTIC CELLS
ADENOVIRUS POUR LE TRANSFERT D'ADN ETRANGER DANS DES CELLULES EUCARIOTES SUPERIEURES

(30) Priorität: 08.04.1993 DE 4311651
(43) Veröffentlichungstag der Anmeldung: 31.01.1996
(73) Patentinhaber: Boehringer Ingelheim International GmbH, 55218 Ingelheim am Rhein (DE); GENENTECH, INC., South San Francisco California 94080 (US)
(72) Erfinder: COTTEN, Matthew, A-1130 Wien (AT); WAGNER, Ernst, A-2103 Langenzersdorf (AT)
(74) Vertreter: Laudien, Dieter, Dr.
(86) Internationale Anmeldenummer: EP9401065
(87) Internationale Veröffentlichungsnummer: WO9424299

(56) Entgegenhaltungen:
- WO-A-92/06180
- WO-A-93/07283
- WO-A-93/09221
- WO-A-94/06923
- WO-A-94/10323
- PROCEEDINGS OF THE NATIONAL ACADEMY OF SCIENCES OF USA. Bd. 89, Nr. 13 , Juli 1992 , WASHINGTON US Seiten 6099 - 6103 WAGNER, E. ET AL. 'Coupling of adenovirus to transferrin-polylysine/DNA complexes greatly enhances receptor-mediated gene delivery and expression of transfected genes'
- PROCEEDINGS OF THE NATIONAL ACADEMY OF SCIENCES OF USA. Bd. 88, Nr. 19 , Oktober 1991 , WASHINGTON US Seiten 8850 - 8854 CURIEL, D.T. ET AL. 'Adenovirus enhencement of transferrin-polylysine-mediated gene delivery' in der Anmeldung erwähnt

## Beschreibung

Die Erfindung bezieht sich auf das Einbringen von Nukleinsäuren in höhere eukaryotische Zellen.

Bedarf an einem effizienten System für das Einführen von Nukleinsäure in lebende Zellen besteht vor allem im Rahmen der Gentherapie. Dabei werden Gene in Zellen eingeschleust, um in vivo die Synthese therapeutisch wirksamer Genprodukte zu erzielen, z.B. um im Falle eines genetischen Defekts das fehlende Gen zu ersetzen.

Für den Transfer von Genen in die Zellen werden z.B. virale Vektoren eingesetzt, die sich der effizienten Eintrittsmechanismen ihrer Ausgangsviren bedienen. Darunter werden Viren verstanden, in deren Genom das in der Zelle zu exprimierende Gen mittels rekombinanter Methoden integriert wurde. Diese Strategie wurde bei der Konstruktion rekombinanter retroviraler und adenoviraler Vektoren angewendet, um einen hoch wirksamen Gentransfer in vitro und *in vivo* zu erzielen. Die bisher am weitesten fortgeschrittenen Technologien für die Anwendung von Nukleinsäuren im Rahmen der Gentherapie benutzen retrovirale Systeme für den Transfer von Genen in die Zelle (Wilson et al., 1990; Kasid et al., 1990). Es wurden daher bereits Methoden entwickelt, um die Anwendbarkeit der retroviralen Systeme zu erweitern bzw. deren Spezifität für eine definierte Zellpopulation zu ermöglichen, indem z.B. der Tropismus der Viren verändert wurde.

Von Roux et al., 1989, und in der Französischen Patentanmeldung 2 649 119 wurde ein System beschrieben, das den Tropismus von Retroviren mit Hilfe bifunktioneller Konjugate verändert, die auf der einen Seite einen Antikörper gegen die Virushülle und auf der anderen Seite einen spezifischen Zellmembranmarker für die Zielzellen enthalten und somit die Verbindung des Virus mit der Wirtszelle herstellen.

Der von Goud et al., 1988, vorgeschlagene Ansatz beruht ebenfalls auf dem Prinzip bifunktioneller Konjugate. Diese Konjugate sind eine Konstruktion aus zwei monoklonalen Antikörpern, von denen einer gegen den humanen Transferrinrezeptor und einer gegen das gp70 Hüllprotein des Moloney-Retrovirus gerichtet ist. Mit Hilfe dieser Konjugate konnte das Retrovirus zwar in die Zielzellen eindringen, nicht jedoch in diesen replizieren.

Die in der WO 92/06180 beschriebene Methode zur Veränderung des Tropismus eines Virus besteht darin, die Oberfläche eines Virus mit einem Molekül zu versehen, das an einen Oberflächenrezeptor der Zielzelle bindet, womit das Virus eine - natürlicherweise nicht vorhandene - Spezifität für die Zielzelle erhält. In der WO 92/06180 wird die Modifikation eines Retrovirus und von Hepatitisvirus B mit Kohlenhydratmolekülen beschrieben, die an den Asialoglykoproteinrezeptor binden.

Für die Anwendung im Rahmen der Gentherapie sind in jüngster Zeit an die Stelle rekombinanter Retroviren zunehmend rekombinante Adenoviren getreten (Berkner, 1988; Stratford-Perricaudet et al., 1990; Rosenfeld et al., 1991; Rosenfeld et al., 1992; Stratford-Perricaudet et al., 1992).

Adenovirale Vektoren haben die vorteilhafte Fähigkeit, in nicht-teilende Zellen eindringen und eine DNA-Fremdsequenz im Ausmaß von bis zu 8.5 kb aufnehmen zu können. Ferner können die Adenoviruspartikel ausgiebig gereinigt werden, ohne an Stabilität einzubüßen, und in Titern höher als 10¹¹PFUs/ml hergestellt werden.

Ein Beschränkung bei der Anwendung der von den Adenoviren Ad2 und Ad5 abgeleiteten rekombinanten Vektoren besteht in ihrer nur geringen Fähigkeit, in Blutzellen einzudringen. Blutzellen sind jedoch ein bevorzugtes Ziel für gentherapeutische Anwendungen, weil sie leicht verfügbar sind und in den Patienten wiedereingeführt werden können, auch sind die Methoden zu ihrer Gewinnung und Kultivierung etabliert. Der Grund für die schwache Aktivität der adenoviralen Vektoren in Blutzellen liegt in der offensichtlich geringen Zahl an Rezeptoren für Adenoviren auf diesen Zellen (Horvath und Weber 1988; Silver und Anderson, 1988). Während die Bindung des Virus an diese Zellen um das Zwei- bis Fünffache reduziert ist, ist die Internalisierung des gebundenen Virus noch wesentlich geringer. Die von vornherein geringe Rezeptorzahl dürfte also mit einer stark verminderten Internalisierung der vorhandenen Rezeptoren einhergehen.

Kürzlich wurde in mehreren Arbeiten der Einsatz von nicht-rekombinanten Adenoviren aufgrund der Fähigkeit von Adenoviren, den Inhalt von Endosomen freisetzen zu können, für den Gentransfer mit DNA-Komplexen mittels Rezeptor-vermittelter Endozytose vorgeschlagen. Der Einsatz von Adenoviren bewirkt eine Steigerung der Effizienz des Gentransfers, indem der Abbau der in die Zelle internalisierten DNA-Komplexe in den Lysosomen vermieden wird (Curiel et al., 1991; Curiel et al., 1992a; Zatloukal et al., 1992; Cotten et al., 1992; Wagner et al., 1992; Curiel et al., 1992b; Yoshimura et al., 1993; WO 93/07283). Es wurde u.a. vorgeschlagen, die Adenoviren durch Bindung an Polylysin zu modifizieren. Die Adenovirus-Polylysin-Konjugate können zusammen mit Konjugaten aus Transferrin-Polylysin mit DNA komplexiert werden, wobei ternäre Transferrin-Polylysin/Adenovirus-Polylysin/DNA-Komplexe entstehen (Wagner et al., 1992). Im Zuge dieser Arbeit wurde festgestellt, daß K562-Zellen in Gegenwart von freiem Adenovirus bei Transfektion mit Transferrin-Polylysin-Konjugaten nur sehr geringe Expressionsraten des importierten Reportergens zeigten, während Polylysingekoppeltes Adenovirus, gemeinsam mit Transferrin-Polylysin unter Bildung eines ternären Komplexes an die Reporter-DNA komplexiert, sehr gute Ergebnisse brachte. Dieses Phänomen ist vermutlich darauf zurückzuführen, daß die Internalisierung der DNA-Komplexe in Blutzellen, die eine geringe Zahl an Adenovirusrezeptoren aufweisen, über den Transferrinrezeptor abläuft, über den die Blutzellen in großer Zahl verfügen.

Adenoviren können nicht in Zellen eindringen, die Adenovirusrezeptoren nicht oder in für eine effiziente Internalisierung ungenügender Zahl aufweisen, oder auch, was vor allem bei Anwendungen *in* vivo von Bedeutung ist, aufgrund der Tatsache, daß die Bindungsstellen des Virus, z.B. durch einen Antikörper, blockiert sind.

Der vorliegenden Erfindung lag die Aufgabe zugrunde, Adenoviren mit der Fähigkeit bereitzustellen, in Zellen, in die sie normalerweise nicht eindringen können, effizient unter Beibehaltung ihrer Fähigkeit zur Genexpression und/oder ihrer endosomolytischen Eigenschaften eindringen zu können.

Die Erfindung betrifft somit ein Virus für den Transport von Fremd-DNA in höhere eukaryotische Zellen, dessen Oberfläche mit einem Liganden für einen Oberflächenrezeptor der Zielzelle derart modifiziert ist, daß es an die Zelle bindet und derart internalisiert wird, daß die Fremd-DNA in der Zelle exprimiert wird. Das Virus ist dadurch gekennzeichnet, daß das Virus ein Adenovirus und der Ligand Transferrin ist, wobei das Transferrin über seine Kohlenhydratseitenketten an die Aminogruppen des Virus gebunden ist.

Es wurde überraschend festgestellt, daß mittels Transferrin-modifiziertem Adenovirus Fremd-DNA in Blutzellen, die der Aufnahme von nicht-modifiziertem Adenovirus nicht zugänglich sind, internalisiert und effizient zur Expression gebracht wird. Bei der Infektion einer Zelle durch ein Virus läuft eine Reihe komplexer Vorgänge ab, die bei den verschiedenen Viren unterschiedlichen Strategien folgen. Da am produktiven Eintritt eines Virus zahlreiche Ereignisse beteiligt sind, die mit der Bindung des Virus an seinen Rezeptor in Zusammenhang stehen, wobei z.B. eine Konformationsänderung, die das Virus bei der Bindung an seinen Rezeptor erfährt, eine für den Ablauf der Internalisierung und Replikation unabdingbare Voraussetzung sein kann, konnte nicht vorausgesagt werden, ob der Zusammenhang der für die Infektion erforderlichen Ereignisse erhalten bleibt, wenn am Virus Modifikationen vorgenommen wurden.

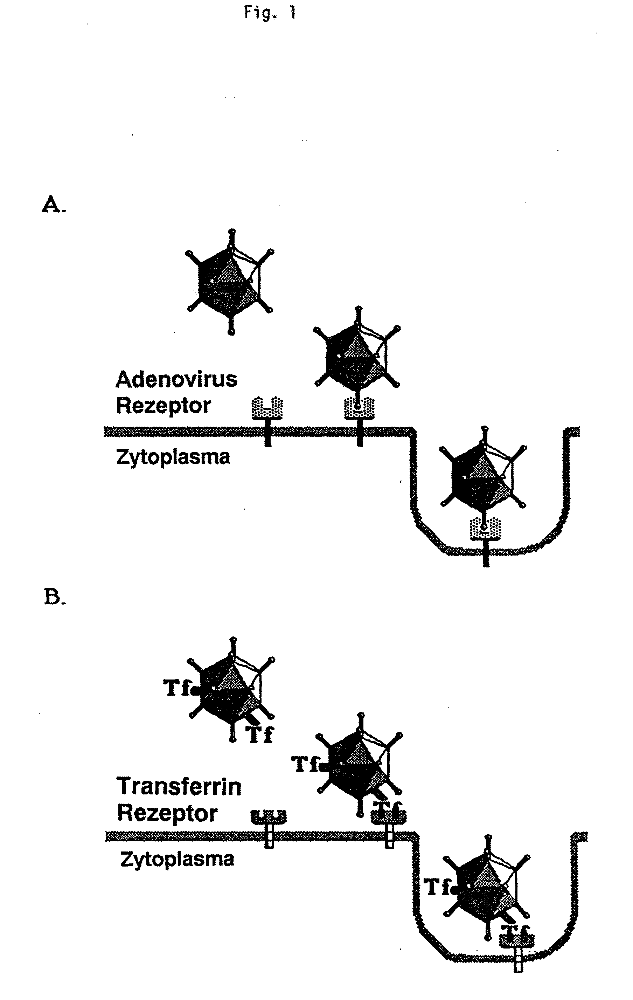

Der Eintritt von Transferrin-modifiziertem Adenovirus über den Transferrinrezeptor (B) im Vergleich zum Eintritt des nicht-modifizierten Virus (A) über seinen Rezeptor ist schematisch in Fig. 1 dargestellt.

Die erfindungsgemäßen Transferrin-modifizierten Adenoviren können verwendet werden, um in trans die Aufnahme von Transferrin-Polylysin/DNA-Komplexen in Zellen zu verbessern, die Transferrin-Rezeptoren, aber keine oder nicht genügend Adenovirus-Rezeptoren aufweisen. Bei der Anwendung *in trans* wird das modifizierte Adenovirus gemeinsam mit Transferrin-Polylysin/DNA-Komplexen auf die zu transfizierenden Zellen aufgebracht, um zusammen mit den DNA-Komplexen in die Zelle aufgenommen zu werden und aufgrund seiner endosomolytischen Fähigkeit die Freisetzung der DNA-Komplexe aus den Endosomen zu bewirken (Curiel et al. 1991).

Das modifizierte Adenovirus kann auch verwendet werden, um als Bestandteil von ternären oder Kombinationskomplexen (Zatloukal et al., 1992) die Effizienz dieser Komplexe zu steigern. Zu diesem Zweck wird das erfindungsgemäße Transferrin-modifizierte Virus, beispielsweise durch eine Biotin-Streptavidin-Brücke, mit einem Polylysin/DNA-Komplex verbunden, wobei gegebenenfalls auch das Polylysin mit Transferrin konjugiert ist. Durch das an das Adenovirus gekoppelte Transferrin übernimmt in diesem Fall das Virus bei Zellen, die Transferrin-Rezeptoren haben, zusätzlich zu seiner endosomolytischen Funktion die Funktion eines Internalisierungsfaktors für den Kombinationskomplex.

Die erfindungsgemäßen Adenovirus-Konjugate können in sämtlichen Anwendungen, in denen Adenoviren eine Steigerung des Gentransfers bewirken, zum Einsatz kommen (Curiel et al., 1991; Curiel et al., 1992a; Zatloukal et al., 1992; Cotten et al., 1992; Wagner et al., 1992; Curiel et al., 1992b; Yoshimura et al., 1993).

In einer Ausführungsform der Erfindung ist das Adenovirus ein rekombinantes Adenovirus, d.h. ein Adenovirus, das in seinem Genom mittels rekombinanter Methoden integrierte Fremdsequenzen enthält. Dabei sind vor allem Sequenzen von Interesse, deren Expression in der Zielzelle einen erwünschten biologischen Effekt erzielt, z. B. DNA, die ein defektes Gen ersetzt. In dieser Ausführungsform bietet die vorliegende Erfindung den Vorteil, den limitierten Tropismus, den die ansonsten erfolgreiche Verwendung rekombinanter Adenoviren für die Gentherapie aufweist, zu beseitigen und das System breiter anwendbar zu machen.

Für die erfindungsgemäßen Virus-Konjugate besteht keinerlei Beschränkung hinsichtlich der Adenoviruskomponente, sämtliche Adenoviren, die an ihrer Oberfläche eine zur Bindung an Transferrin fähige Gruppierung aufweisen, sind geeignet, z.B. die von Berkner, 1988; Stratford-Perricaudet et al., 1990; Rosenfeld et al., 1991; Rosenfeld et al., 1992; Stratford-Perricaudet et al., 1992, beschriebenen Adenoviren, die als Vektoren für den Import von DNA in die menschliche Zelle, insbesondere für die gentherapeutische Anwendung, vorgeschlagen wurden, können modifiziert werden, um DNA selektiv in *vivo* oder *ex vivo* in die Zielzelle zu befördern.

Im Hinblick auf die Anwendung im Menschen wird als Transferrin-Komponente insbesondere Humantransferrin verwendet. Transferrin ist ein Eisentransportprotein, das mit hoher Effizienz über Rezeptor-vermittelte Endozytose in die Zelle aufgenommen wird, wodurch es als Transport-Vehikel bereits für verschiedene Anwendungen benutzt wurde, z.B um in Form verschiedener Konjugate Toxine, niedermolekulare Substanzen oder Gene in die Zelle zu transportieren. Der Vorgang, der bei der Internalisierung von Transferrin durch seinen Rezeptor abläuft, unterscheidet sich von anderen Ligand/Rezeptor-Paarungen u.a. dadurch, daß der Transferrinrezeptor mit hohem Durchsatz rezykliert wird.

Bezüglich der Fremd-DNA bestehen keinerlei durch die vorliegende Erfindung bedingten Beschränkungen; die DNA kann ein beliebiges Gen sein oder auch ein Plasmid-Konstrukt, das z.B. für inhibierende RNA kodierende Elemente enthält. Gentherapeutisch wirksame Sequenzen sind dem Fachmann bekannt; Beispiele für derzeit als für therapeutisch aussichtsreich angesehene Sequenzen sind u.a. der Übersicht von Anderson, 1992, entnehmbar.

Die Erfindung betrifft in einem weiteren Aspekt ein Verfahren zum Einbringen von Fremd-DNA in menschliche Zellen, die keine oder nur eine geringe Zahl von Adenovirus-Rezeptoren aufweisen oder deren Adenovirus-Rezeptoren ganz oder teilweise blockiert sind, in welchem man die Zellen mit dem erfindungsgemäßen Virus in geeigneter Formulierung ex vivo oder in vivo behandelt.

Insbesondere sind die Zellen Blutzellen.

Die Anforderungen an die Formulierung, in der die erfindungsgemäßen modifizierten Viren verabreicht werden, werden durch die spezielle Anwendung definiert; der Fachmann kann einschlägigen pharmazeutischen Handbüchern (z.B. Remington's Pharmaceutical Sciences, 1980) zahlreiche Träger- und Zusatzstoffe entnehmen, die für die Formulierung verwendet werden, wesentlich ist vor allem, daß die Formulierung die Transport-Funktion des erfindungsgemäßen Virus-Transferrin-Konjugats sowie die Bioverfügbarkeit des in der Zelle exprimierten Proteins nicht beeinträchtigen.

Das Virus kann an Transferrin in für die Kopplung von Peptiden an sich bekannter Weise erfolgen, vorzugsweise erfolgt die Bindung des Adenovirus an Transferrin über die Kohlenhydratseitenketten des Transferrins. Dieser Typ Bindung ist in der DE-A1 41 150 38, auf deren Offenbarung hiermit Bezug genommen wird, für Transferrin-Polylysin-Konjugate beschrieben.

Es wurde überraschend festgestellt, daß sich diese Art der Bindung sehr gut zur Modifikation von Adenoviren mit Transferrin eignet, um Fremd-DNA in Zellen zu importieren, die ansonsten dem Transportvehikel Adenovirus nicht bzw. nicht ausreichend zugänglich sind.

Voraussetzung für die Fähigkeit des Virus, an die Kohlenhydratseitenketten des Transferrins zu koppeln, ist das Vorliegen von Aminogruppen an der Oberfläche des Virus. Ohne auf diese Theorie festgelegt sein zu wollen, dürfte es dabei von Vorteil sein, daß die Kohlenhydratseitenkette des Transferrins einen natürlichen Abstandhalter zwischen Transferrin und dem Virus darstellt. Dadurch dürfte einerseits die Bindungs- und Internalisierungsfähigkeit des Transferrins, andererseits dürfte, was für die Anwendung der erfindungsgemäßen Konjugate als Bestandteil von Kombinationskomplexen von Bedeutung ist, die endosomolytische Aktivität des Adenovirus erhalten bleiben.

Ein bevorzugtes Verfahren zur Herstellung der erfindungsgemäßen Adenovirus-Transferrin-Konjugate, das ebenfalls Gegenstand der vorliegenden Erfindung ist, besteht darin, Transferrin unter schonenden Bedingungen zu einer im Kohlenhydratanteil Aldehydgruppen enthaltenden Form zu oxidieren und das oxidierte Transferrin unter reduzierenden Bedingungen mit dem Adenovirus zu koppeln.

Für den Oxidationsschritt, bei dem terminale Sialinsäuren der Kohlenhydratketten des Transferrins zur Aldehydform oxidiert werden, wird als Oxidationsmittel bevorzugt Perjodat, insbesondere Natriumperjodat, eingesetzt.

Als Substanzen zur Schaffung reduzierender Bedingungen bei der Kopplung der Aldehydform des Transferrins mit dem Adenovirus sind Reduktionsmittel geeignet, die unter schonenden Bedingungen Schiff'sche Basen selektiv reduzieren. Bevorzugt für den Einsatz als Reduktionsmittel im erfindungsgemäßen Verfahren sind Natriumcyanoborhydrid oder tertiäres Butylaminboran.

Das Verfahren wird bevorzugt bei niedrigen Temperaturen, insbesondere 0°C bis Raumtemperatur, durchgeführt.

Die Mechanismen der einzelnen Reaktionsstufen sind dem Durchschnittsfachmann geläufig; es liegt daher im Rahmen seiner Fähigkeiten, die Bedingungen für die einzelnen Verfahrensschritte den jeweiligen individuellen Bedürfnissen anzupassen. In einzelnen Anwendungsfällen kann es erstrebenswert sein, das Viruspartikel mit einer geringeren Anzahl von Transferrinmolekülen auszustatten als in den im Rahmen der vorliegenden Erfindung durchgeführten Versuchen, um ein optimales Gleichgewicht zwischen Internalisierungs- und endosomolytischer Eigenschaft zu erhalten. Modifizierte Adenoviruspartikel mit unterschiedlichen Anteilen von daran gebundenem Transferrin können erhalten werden, indem das Verhältnis Adenovirus/Reagentien empirisch verändert wird.

### Figurenübersicht:

- Fig. 1:: Schematische Darstellung des Viruseintritts in die Zelle. A: Nicht-modifiziertes Adenovirus.
B: Transferrin-modifiziertes Adenovirus.
- Fig. 2:: Quantifizierung des an Adenovirus gebundenen Transferrin auf Nitrozellulosemembranblot
- Fig. 3:: Expression von β-Galaktosidase in K562-Zellen nach Transfektion mit rekombinantem Adenovirus. A: Nicht-modifiziertes Adenovirus.
B: Transferrin-modifiziertes Adenovirus. C: Kontrolle (nicht-infizierte Zellen).
- Fig. 4:: Quantitativer Vergleich mittels Luminometrie des Gentransfers in K562-Zellen

Die Erfindung wird anhand des folgenden Beispiels illustriert:

### Beispiel

### a) Adenoviruspräparation

5 x 10⁶ 293-Zellen (ATCC No. 1573; Graham et al., 1977) wurden in 175 cm² Flaschen mit 60 ml DMEM Medium plus 10 % FCS und 1 % Glutamin sowie einem Antibiotikazusatz (Penicillin, Streptomycin) 3 Tage bei 37°C und 5 % CO₂ gezüchtet, bis ca. 2 x 10⁷ Zellen, die zu ca. 80 - 100 % konfluent waren, erhalten wurden. Danach wurde das Medium entfernt und die Zellen mit Adenovirus Ad.RSVβgal (E1-, E3-defektes Adenovirus Typ 5, das das E.coli β-Galaktosidasegen unter Kontrolle des RSV Promotor/Enhancers trägt; Stratford-Perricaudet et al., 1992) infiziert (ca. 2 x 10⁹ Partikel in 5 ml Medium mit 2 % FCS). Nach ca. 2 Tagen Inkubation bei 37°C waren die Zellen angeschwollen und hatten sich nahezu vollständig vom Boden abgelöst. Daraufhin wurden die Zellen 10 min in einem Sorvall GSA Rotor bei 3.000 rpm zentrifugiert, die Zellpellets mit PBS in ein 50 ml Röhrchen transferiert und 10 min in einer Heraeus Zentrifuge bei 1.000 rpm zentrifugiert. Das Pellet wurde im 2- bis 3fachen Volumen 10 mM HEPES/1 mM EDTA (HE) aufgenommen, in flüssigem Stickstoff schockgefroren und bei -70°C gelagert. Für den Zellaufschluß wurden die Zellen vier Frier-/Tauzyklen (flüssiger Stickstoff/37°C Wasserbad) unterworfen und 10 min zentrifugiert (Heraeus, 4.000 rpm). Anschließend wurde das Zellysat einer Ultrazentrifugation unterworfen (vTI-50 47.000 rpm/l h/20°C; CsCl Dichtegradient: 20 ml 1.33 g/cm³ CsCl in HE, unterschichtet mit 10 ml 1.45 g/cm³ CsCl in HE, überschichtet mit 10 ml Zellysat). Die opaleszente Virusbande wurde gesammelt und einer zweiten Ultrazentrifugation unterworfen (vTI-50 63.000 rpm/ 4 h/20°C; CsCl Gleichgewichtsgradient: 2.5 ml 1.33 g/cm³ CsCl in HE, gemischt mit 2.5 ml Virusbande). Pro Zellkulturflasche wurden 1 - 3 x 10¹¹ Viruspartikel erhalten, die mit 40 % (v/v) Glycerin bei -70°C gelagert wurden. Die Bestimmung der Virion-Konzentration wurde über den Proteingehalt mittels Bradfordassay (BSA, Fraktion V, BMB als Standard) bestimmt, wobei das Verhältnis 1 mg/ml Virusprotein = 1.34 x 10¹² Viruspartikel (Lemay et al., 1980) herangezogen wurde.

### b) Modifikation des Adenovirus mit Transferrin

Eine Lösung von 105 mg (1.30 µmol) Transferrin (human, Sigma) in 1 ml 30 mM Natriumacetatpuffer (pH 5) wurde einer Gelfiltration auf einer Sephadex G-25 PD10 Säule (Pharmacia) unterworfen, wobei derselbe Puffer verwendet wurde. Die erhaltenen 2 ml Lösung, die 80 mg (1.0 µmol) Transferrin enthielten (der Transferringehalt wurde durch UV-Messung bei 280 nm und Ninhydrinassay bestimmt), wurden in einem Speedvac (Savant) auf 1 ml konzentriert, auf 0°C gekühlt und mit 50 µl 30 mM Natriumacetatpuffer (pH 5), enthaltend 1.1 mg (5 µmol) Natriumperjodat, behandelt. Die Mischung wurde in einem Eisbad im Dunklen 90 min lang stehen gelassen. Anschließend wurde eine weitere Gelfiltration (Sephadex G-25 PD10 Säule, Pharmacia, 150 mM NaCl, 10 mM HEPES, pH 7.3) durchgeführt, dabei wurden 1 ml einer Lösung, enthaltend 66 mg (0.82 µmol) oxidiertes Transferrin erhalten (der Gehalt an oxidierter, Aldehyd enthaltender Form von Transferrin wurde bestimmt mittels Färbung mit Anisaldehydreagens, wie von Wagner et al., 1991, beschrieben). Ein Teil der modifizierten Transferrinlösung (0.6 ml; 0.5 µmol) wurde rasch zu einer Lösung, enthaltend 25 µg (bezogen auf Protein) Adenovirus in 300 µl HBS, hinzugefügt. Nach 1 h bei Raumtemperatur wurde eine Lösung von 1 mg (15 µmol) Natriumcyanborhydrid zugegeben. Nach 24 h bei Raumtemperatur wurde das Transferrin-konjugierte Adenovirus von überschüssigem freiem Transferrin gereinigt, indem das Virus mit einem gleichen Volumen HBS (150 mM NaCl, 20 mM HEPES, pH 7.4) verdünnt und das Material über einen CsCl-Stufengradienten zentrifugiert wurde. Ca. 1.5 ml des modifizierten Virus wurden in einem vTI-65 Röhrchen (Beckman) mit 3 ml 1.31 g/cm³ CsCl und 1 ml 1.45 g/cm³ CsCl (beide CsCl-Lösungen in 1 mM EDTA, 20 mM HEPES, pH 7.4) unterschichtet. Die Probe wurde 1 h lang bei 63.000 rpm in einem vTi-65 Rotor (Beckman) zentrifugiert. Die opaleszente Virusbande wurde geerntet, mit einem gleichen Volumen HBS verdünnt und einer zweiten identischen CsCl-Gradientenreinigung unterworfen. Alternativ wurde die erste Virusbande mit 1.33 g/cm³ CsCl, 20 mM HEPES, 1 mM EDTA, pH 7.4, auf 5.5 ml gebracht und die Probe bis zum Gleichgewicht zentrifugiert (4 h, 63.000 rpm, vTi-65 Rotor). Das gereinigte modifizierte Virus wurde geerntet, mit einem gleichen Volumen von 96 % Glyzerin (Fluka) verdünnt und bis zur Verwendung bei -70°C gelagert. Parallel wurde eine Kopplung mit eisenfreiem Transferrin durchgeführt; das gereinigte Virus wurde mit 1 µl 1 mM Eisen (III) Citratpuffer, pH 7.5 versetzt.

### c) Quantitative Bestimmung des an Adenovirus gebundenen Transferrins

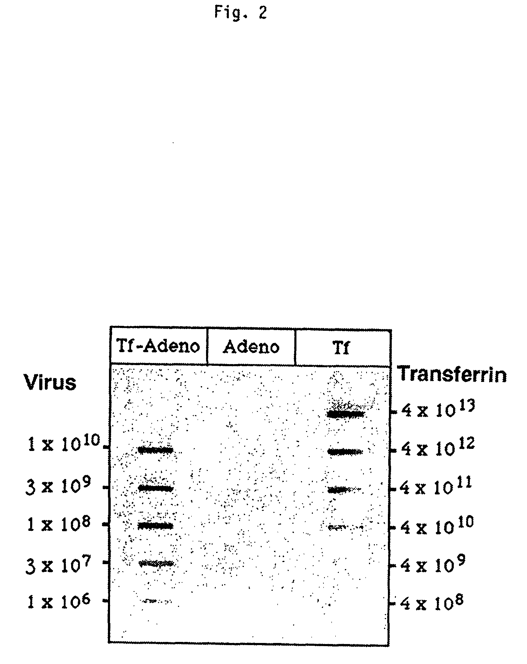

Serienverdünnungen von Transferrin-modifiziertem Adenovirus, nicht-modifiziertem Adenovirus sowie Transferrin-Standards (jeweils in HBS; die Anzahl der aufgetragenen Viruspartikel bzw. Transferrinmoleküle ist Fig. 2 entnehmbar) wurden an eine Nitrozellulosemembran (Schleicher & Schuell, 0.1 mm Porengröße) gebunden. Der Blot wurde über Nacht bei 4°C mit 3 % (w/v) pulverisierter Magermilch in HBS (10 ml) vorhybridisiert. Der Transferringehalt jeder Probe wurde bestimmt, indem die Membran 4 h bei Raumtemperatur einem Mausantikörper, der humanes Transferrin erkennt (Chemicon MAB 033-19/1, Verdünnung 1:2000 in HBS/Milch, 10 ml), und im Anschluß daran (nach 2 stündigem Waschen mit 4 x 130 ml HBS/Milch) 1 h lang einem ¹²⁵I-markiertem zweiten Antikörper (Schafanti-Maus-Ig, Amersham, Kat.No. L52215, 2 µCi, in 20 ml HBS/Milch) exponiert wurde. Die Phosphoimager-Analyse der Membran zeigt die Gegenwart von Transferrin in den Proben mit modifiziertem Adenovirus, jedoch kein Signal beim nicht-modifizierten Adenovirus. Der Vergleich der mit dem modifizierten Virus erhaltenen Signale mit den Transferrinstandards erlaubt eine Bestimmung der Menge an Transferrin, die an ein Virusteilchen gebunden ist. Es wurde gefunden, daß 3 x 10⁹ Viruspartikel ein Signal geben, das mit dem von 4 x 10¹² Transferrinmolekülen vergleichbar ist. Daraus ergibt sich, daß an ein Adenovirusteilchen ca. 1.000 Transferrinmoleküle gebunden sind. Die Assoziation der Transferrinmoleküle mit Adenovirusteilchen durch zwei CsCl-Dichtegradienten spricht für eine kovalente Bindung zwischen Kapsidproteinen des Virus und dem Transferrinmolekül. Damit in Einklang ist auch das Ergebnis einer Analyse von Viruskapsidproteinen mittels SDS-PAGE, die zeigte, daß die Hauptmenge des gekoppelten Transferrins an das Hexon gebunden ist.

### d) Transfektion von K562-Zellen mit Transferrin-modifiziertem rekombinantem Adenovirus, enthaltend als Fremd-DNA das β-Galaktosidasegen

K562-Zellen (ATCC No. CCL 243) wurden in Suspension in RPMI 1640 Medium (Gibco BRL) plus 10 % FCS, 100 Einheiten/ml Penicillin, 100 µg/ml Streptomycin und 2 mM Glutamin gezüchtet. 20 h vor der Transfektion wurden die Zellen in frisches Medium, enthaltend zwecks Erhöhung ihrer Transferrinrezeptor-Zahl 50 µM Deferrioxamin (Cotten et al., 1990), überführt. Die Transfektionen wurden bei einer Dichte von 250.000 Zellen/ml in demselben Medium (plus 50 µM Deferrioxamin) durchgeführt. Es wurden jeweils gleiche Mengen (3.000 Viruspartikel/Zelle bis 300 Viruspartikel/Zelle) von entweder nicht-modifiziertem Adenovirus (Ad.RSVβgal) oder von modifiziertem Adenovirus (Tf-Ad.RSVβgal) auf die K562-Zellen aufgebracht. 48 h nach der Transfektion wurden ca. 25.000 Zellen in einem Volumen von 100 bis 200 µl in die Vertiefungen einer rundbödigen 96-Well-Platte überführt und bei 800 rpm in einem Beckman GH 3.7 Rotor 10 min lang zentrifugiert. Der Überstand wurde vorsichtig entfernt und mit 100 µl 0.5 % Glutardialdehyd in HBS ersetzt. Die Zellen wurden durch Pipettieren dispergiert und dann wieder zentrifugiert. Daraufhin wurde das Fixiermittel entfernt und die Zellen gewaschen. Anschließend wurde mit 200 µl Färbelösung (10 mM Phosphatpuffer pH 7.0, 150 mM NaCl, 1 mM MgCl₂, 3.3 mM K₄Fe(CN)₆3H₂O, 3.3 mM K₃Fe(CN)₆ und 0.2 % 5-Brom-4-chlor-3-indolyl-β-galaktopyranosid) bei 37 °C 20 min bis 3 h inkubiert (Lim und Chae, 1989). Die Zellen wurden bei 38°C 5 h lang inkubiert, dann 2 x gewaschen und nach dem Transfer in flachbödige Vertiefungen einer 96-Well-Platte photographiert.

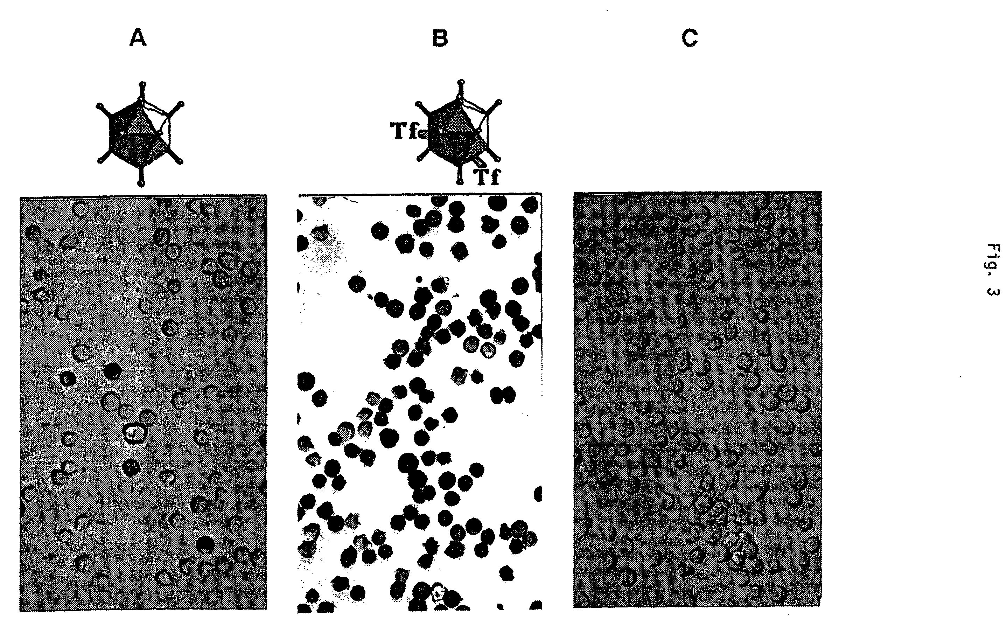

Das Ergebnis der Transfektionen ist in Fig. 3 wiedergegeben: Es wurde gefunden, daß beim höchsten Virus/Zellverhältnis das nicht-modifizierte Virus die Zellen mit einer Leistungsfähigkeit von ca. 5 % transduzieren kann. Beim selben Virus/Zellverhältnis transduziert jedoch das Transferrin-modifizierte Virus mehr als 90 % der Zellpopulation, wobei auch die tatsächlichen Mengen an pro Zelle produzierter β-Galaktosidase höher sind. Kontrollversuche zeigten, daß sowohl nicht-modifiziertes als auch Transferrin-modifiziertes Adenovirus in HeLa-Zellen mit gleicher Leistungsfähigkeit eindringen konnte.

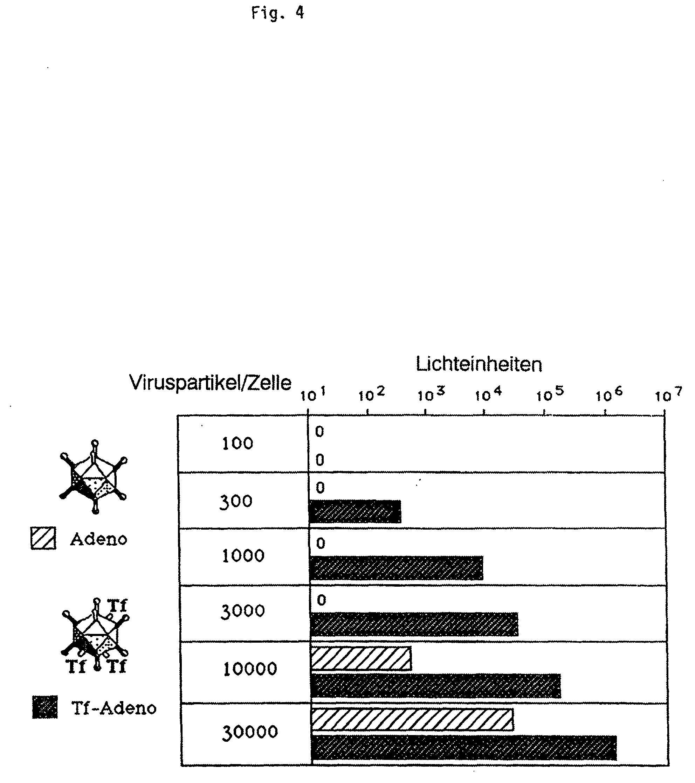

Zusätzlich wurde eine Analyse der β-Galaktosidase-Aktivität mittels Luminometrie nach der von Jain und Magratz, 1991, beschriebenen Methode durchgeführt. Dazu wurden K562-Zellen 18 h lang in RPMI/10 % FCS, enthaltend 50 uM Deferrioxamin gezüchtet. Unmittelbar vor der Infektion wurden die Zellen in frisches Deferrioxamin enthaltendes Medium gegeben (250.000 Zellen/ml, 50.000 Zellen pro Vertiefung einer Platte mit 96 Vertiefungen). Die Verdünnungen der Kontrollen und der Proben mit Transferrin-modifiziertem Adenovirus Ad.RSVβgal wurden in RPMI/2 % hitzeinaktiviertem Pferdeserum hergestellt und Aliquots des Virus (50 µl), die die in der Fig. 4 angegebene Anzahl an Viruspartikeln pro Zelle enthielten, auf die Zellen aufgegeben. Nach 4 h bei 37°C wurden die Zellen in frischem Medium (ohne Deferrioxamin) gewaschen. Nach 24 h bei 37°C wurden Aliquots von 50.000 infizierten oder Kontrollzellen zur luminometrischen Messung mittels Zentrifugation gesammelt, in 100 µl 0.25 M Tris pH 7.5 aufgenommen und durch drei Frier/Auftauzyklen (flüssiger Stickstoff/37°C) aufgebrochen. Die Zelltrümmer wurden durch Zentrifugieren entfernt (14.000 x g, Eppendorf), und Aliquots des Überstandes, auf Proteingehalt standardisiert, wurden unter Verwendung des chemilumineszierenden Substrats AMPGD und einem Emerald Luminiszenzverstärker (Tropix) analysiert. Das Ergebnis der Messungen ist in Fig. 4 dargestellt.

### Literatur

Anderson, F.W. , 1992, Science 256, 808.
Berkner, K.L., 1988, BioTechniques 6, 616-629.
Cotten, M., Wagner, E., Zatloukal, K., Phillips, S., Curiel, D.T. und Birnstiel, M.L., 1992, Proc.Natl.Acad.Sci. USA 89, 6094-6098.
Cotten, M., Laengle-Rouault, F., Kirlappos., H., Wagner, E., Mechtler, K., Zenke, M., Beug, H., und Birnstiel, M.L., 1990, Proc.Natl.Acad.Sci. USA 87, 4033-4037.
Curiel, D.T., Agarwal, S., Wagner, E. und Cotten, M., 1991, Proc.Natl.Acad.Sci. USA 88, 8850-8854.
Curiel, D.T., Agarwal, S., Romer, M.U., Wagner, E., Cotten, M., Birnstiel, M.L. und Boucher, R.C., 1992a, Am.J.Respir.Cell and Mol.Biol. 6, 247-252.
Curiel, D.T., Wagner, E., Cotten, M., Birnstiel, M.L., Agarwal, S., Li, Ch.-M., Loechel, S. und Hu, P.-H., 1992b, Human Gene Therapie 3, 147-154.
Goud, B., Legrain, P. und Buttin, G., 1988, Virology 163, 251-254.
Graham, F., Smiley, J., Russel, W.C. und Nairu, R., 1977, J. Gen. Virol. 36, 59-72.
Horvath, J. und Weber, J., 1988, J. Virol. 62, 341-345. Jain, V.K. und Magrath, I.T., 1992, Anal. Biochem. 199, 119-124.
Kasid, A., Morecki, S., Aebersold, P., Cornetta, K., Culver, K., Freeman, S., Director, E., Lotze, M.T., Blaese, R.M., Anderson, W.F. und Rosenberg, S.A., 1990, Proc.Natl.Acad.Sci. USA 87, 473-477.
Lemay, P., Boudin, M., Milleville, M. und Boulanger, P., 1980, Virology 101, 131-143.
Lim, K. und Chae, C.B., 1989, BioTechniques 7, 576-579. Rosenfeld, M.A., Siegfried, W., Yoshimura, K., Yoneyama, K., Fukayama, M., Stier, L.E., Paakko, P.K., Gilardi, P., Stratford-Perricaudet, L.D., Perricaudet, M. et al., 1991, Science 252, 431-434.
Rosenfeld, M.A., Yoshimura, K., Trapnell, B., Yoneyama, K., Rosenthal, E., Dalemans, W., Fukayama, M., Bargon, J., Stier, L., Stratford-Perricaudet, L.D. et al., 1992, Cell 68, 143-155.
Roux, P., Jeanteur, P. und Piechaczyk, M., 1989, Proc.Natl.Acad.Sci. USA 86, 9079-9083.
Stratford-Perricaudet, L., Levrero, M., Chasse, J.-F., Perricaudet, M. und Briand, P., 1990, Hum. Gene Ther. 1, 241-256.
Stratford-Perricaudet, L., Makeh, I., Perricaudet, M. und Briand, P., 1992, J. Clin. Invest. 90, 626-630.
Wagner, E., Cotten, M., Mechtler, K., Kirlappos, H. und Birnstiel, M.L., 1991, Bioconjugate Chemistry 2, 226-231.
Wagner, E., Zatloukal, K., Cotten, M., Kirlappos, H., Mechtler, K., Curiel, D.T. und Birnstiel, M.L., 1992, Proc.Natl.Acad.Sci. USA 89, 6099-6103.
Wilson, J.M., Danos, O., Grossman, M., Raulet, D.H. und Mulligan, R.C., 1990, Proc.Natl.Acad.Sci. USA 87, 439-443.
Yoshimura, K., Rosenfeld, M.A., Seth, P. und Cyrstal, R.G., 1993, J. Biol. Chem. 288, 2300-2303.
Zatloukal, K., Wagner, E., Cotten, M., Phillips, S., Plank, C., Steinlein, P., Curiel, D. und Birnstiel, M.L., 1992, Ann.New York Acad.Sci. 660, 136-153.
Remington's Pharmaceutical Sciences, 1980, Mack Publ. Co., Easton, PA, Osol (ed.).

## Patentansprüche

1. Virus für den Transport von Fremd-DNA in höhere eukaryotische Zellen, dessen Oberfläche mit einem Liganden für einen Oberflächenrezeptor der Zielzelle derart modifiziert ist, daß es an die Zelle bindet und derart internalisiert wird, daß die Fremd-DNA in der Zelle exprimiert wird, **dadurch gekennzeichnet, daß** das Virus ein Adenovirus und der Ligand Transferrin ist, wobei das Transferrin über seine Kohlenhydratseitenketten an die Aminogruppen des Virus gebunden ist.

2. Modifiziertes Virus nach Anspruch 1, **dadurch gekennzeichnet, daß** das Virus ein Adenovirus vom Typ 2 ist.

3. Modifiziertes Virus nach Anspruch 1, **dadurch gekennzeichnet, daß** das Virus ein Adenovirus vom Typ 5 ist.

4. Modifiziertes Virus nach einem der vorhergehenden Ansprüche, **dadurch gekennzeichnet, daß** das Virus ein rekombinantes Adenovirus ist, das als Fremd-DNA eine gentherapeutisch wirksame DNA-Sequenz enthält.

5. Modifiziertes Virus nach einem der Ansprüche 1 bis 4, **dadurch gekennzeichnet, daß** das Transferrin Humantransferrin ist.

6. Verfahren zur Herstellung von Adenovirus-Transferrin-Konjugaten nach Anspruch 1, **dadurch gekennzeichnet, daß** man Transferrin unter schonenden Bedingungen zu einer im Kohlenhydratanteil Aldehydgruppen enthaltenden Form oxidiert und das oxidierte Transferrin unter reduzierenden Bedingungen mit dem Adenovirus koppelt.

7. Verfahren nach Anspruch 6, **dadurch gekennzeichnet, daß** man das Transferrin mit Periodat oxidiert.

8. Verfahren nach Anspruch 6 oder 7, **dadurch gekennzeichnet, daß** man die Kopplung unter reduzierenden Bedingungen in Gegenwart einer Substanz durchführt, die unter für das Transferrin schonenden Bedingungen Schiff'sche Basen selektiv reduziert.

9. Verfahren nach Anspruch 8, **dadurch gekennzeichnet, daß** man die Kopplung in Gegenwart von Natriumcyanoborhydrid vornimmt.

10. Komplex zum Einführen von Fremd-DNA in humane Zellen, **dadurch gekennzeichnet, daß** er Polylysin enthält, das mit einem in einem der Ansprüche 1 bis 3 und 5 definierten Virus sowie gegebenenfalls zusätzlich mit Transferrin konjugiert und mit der Fremd-DNA komplexiert ist.

## Claims

1. Virus for delivering foreign DNA into higher eukaryotic cells, the surface of which is modified with a ligand for a surface receptor of the target cell in such a way that it binds to the cell and is internalised in such a way that the foreign DNA is expressed in the cell, **characterised in that** the virus is an adenovirus and the ligand is transferrin, the transferrin being bound via its carbohydrate side chains to the amino groups of the virus.

2. Modified virus according to claim 1, **characterised in that** the virus is an adenovirus of Type 2.

3. Modified virus according to claim 1, **characterised in that** the virus is an adenovirus of Type 5.

4. Modified virus according to one of the preceding claims, **characterised in that** the virus is a recombinant adenovirus which contains as foreign DNA a DNA sequence which is effective in gene therapy.

5. Modified virus according to one of claims 1 to 4, **characterised in that** the transferrin is human transferrin.

6. Process for preparing adenovirus-transferrin conjugates according to claim 1, **characterised in that** transferrin is oxidised under mild conditions to a form which contains aldehyde groups in the carbohydrate moiety and the oxidised transferrin is coupled with the adenovirus under reducing conditions.

7. Process according to claim 6, **characterised in that** the transferrin is oxidised with periodate.

8. Process according to claim 6 or 7, **characterised in that** the coupling is carried out under reducing conditions in the presence of a substance which selectively reduces Schiff's bases under conditions which are mild for transferrin.

9. Process according to claim 8, **characterised in that** the coupling is carried out in the presence of sodium cyanoborohydride.

10. Complex for introducing foreign DNA into human cells, **characterised in that** it contains polylysine which is conjugated with a virus as defined in one of claims 1 to 3 and 5 and optionally also with transferrin and is complexed with the foreign DNA.

## Revendications

1. Virus pour le transport d'ADN étranger dans des cellules eucaryotiques supérieures, dont la surface est modifiée avec un ligand pour un récepteur de surface de la cellule cible de sorte qu'il se lie à la cellule et est internalisé de sorte que l'ADN étranger est exprimé dans la cellule, **caractérisé en ce que** le virus est un adénovirus et le ligand est la transferrine, la transferrine étant liée aux groupes amino du virus par ses chaînes latérales glucidiques.

2. Virus modifié selon la revendication 1 **caractérisé en ce que** le virus est un adénovirus de type 2.

3. Virus modifié selon la revendication 1 **caractérisé en ce que** le virus est un adénovirus de type 5.

4. Virus modifié selon l'une des revendications précédentes **caractérisé en ce que** le virus est un adénovirus recombiné qui contient comme ADN étranger une séquence d'ADN efficace du point de vue de la thérapie génique.

5. Virus modifié selon l'une des revendications 1 à 4 **caractérisé en ce que** la transferrine est la transferrine humaine.

6. Procédé de préparation de conjugués adénovirus-transferrine selon la revendication 1 **caractérisé en ce que** l'on oxyde la transferrine dans des conditions douces en une forme contenant des groupes aldéhyde dans la partie glucidique et on couple la transferrine oxydée avec l'adénovirus dans des conditions réductrices.

7. Procédé selon la revendication 6 **caractérisé en ce que** l'on oxyde la transferrine avec le periodate.

8. Procédé selon la revendication 6 ou 7 **caractérisé en ce que** l'on réalise le couplage dans des conditions réductrices en présence d'une substance qui réduit sélectivement les bases de Schiff dans des conditions douces pour la transferrine.

9. Procédé selon la revendication 8 **caractérisé en ce que** l'on réalise le couplage en présence de cyanoborohydrure de sodium.

10. Complexe pour l'introduction d'ADN étranger dans des cellules humaines **caractérisé en ce qu'**il contient de la polylysine qui est conjuguée avec un virus défini dans l'une des revendications 1 à 3 et 5 et éventuellement en outre avec la transferrine et qui est complexée avec l'ADN étranger.
